# EUROPÄISCHE PATENTSCHRIFT

(11) **EP 0 599 344 B1**
(45) Veröffentlichungstag und Bekanntmachung des Hinweises auf die Patenterteilung: **24.09.1997**
(21) Anmeldenummer: 93119120.9
(22) Anmeldetag: 26.11.1993
(51) Int. Cl.: C07K 1/113, C12N 9/96

(54) **Verfahren zur Stabilisierung von Proteinen**
Process for the stabilization of proteins
Procédé pour la stabilisation des protéines

(30) Priorität: 27.11.1992 DE 4239969
(43) Veröffentlichungstag der Anmeldung: 01.06.1994
(73) Patentinhaber: BOEHRINGER MANNHEIM GMBH, 68305 Mannheim (DE)
(72) Erfinder: Jakob, Ursula, D-93053 Regensburg (DE); Buchner, Johannes, Dr., D-93346 Ihrlerstein (DE); Gaestel, Matthias, Dr., D-10365 Berlin (DE); Ambrosius, Dorothee, Dr., D-82393 Iffeldorf (DE); Rudolph, Rainer, Prof., D-82362 Weilheim (DE)
(74) Vertreter: Böhm, Brigitte, Dipl.-Chem. Dr.

(56) Entgegenhaltungen:
- PLANT CELL PHYSIOL. Bd. 30, Nr. 4, 1989, Seiten 463-469, JINN T.L. et al.
- J. CELL. BIOL. Bd. 109, Juli 1989, Seiten 7-15, LANDRY J. et al.
- J. BIOL. CHEM. Bd. 268, Nr. 2, 15. Januar 1993, Seiten 1046-1052, MERCK K.B. et al.
- J. BIOL. CHEM. Bd. 268, Nr. 3, 25. Januar 1993, Seiten 1517-1520, JAKOB U. et al.
- ANNU. REV. GENET. Bd. 22, 1988, Seiten 631-677, LINDQUIST S. et al.
- CURR. OPIN. BIOTECH. Bd. 2, 1991, Seiten 532-538, BUCHNER J. et al.

## Beschreibung

Die vorliegende Erfindung betrifft ein Verfahren zur Stabilisierung von Proteinen in einer wäßrigen Lösung, durch Zusatz einer als Stabilisator wirkenden Proteinkomponente.

Bei der Familie der kleinen Hitzeschock-Proteine (kleine Hsp's) handelt es sich um eine heterogene Gruppe von Proteinen mit einem oder mehreren Vertretern in Hefen (Hsp26), Vertebraten (Hsp24, Hsp28), Drosophila (Hsp22, 23, 26, 28), Maus (Hsp25) und über 20 Proteinen in Pflanzen (Nover & Scharf, Eur.J.Biochem. 139 (1984), 303-313; Lindquist & Craig, Ann.Rev.Genet. 22 (1988), 631-677). Ihre Molekularmassen liegen je nach Organismus zwischen 15 und 40 kDa, wobei in den meisten Zellen und Geweben die kleinen Hsp's auch in Abwesenheit von Streß synthetisiert werden und als cytosolische Aggregate (Hitzeschock-Granula) mit einer Molekularmasse von über 700 kDa vorliegen (Arrigo et al., Mol.Cell Biol. 8 (1988), 5059-5071). Hitzeschock und chemischer Streß induzieren die Synthese der kleinen Hsp's, die dann bis zu 1 % des Gesamtzellproteins ausmachen können (Österreich et al., Biomed.Biochim.Acta 49 (1990), 219-226). Zudem bewirken die oben genannten Streßfaktoren eine Veränderung im Phosphorylierungsgrad der Proteine sowie in ihrer zellulären Lokalisation (Arrigo et al., supra; Zantema et al., J.Biol.Chem. 267 (1992), 12936-12941). Die kleinen Hsp's der verschiedenen Organismen zeigen trotz ihrer Heterogenität übereinstimmende Hydrophobizitätsprofile und kleine Regionen identischer Aminosäuresequenzen (Lindquist & Craig, supra). Daneben konnten eindeutige Sequenzhomologien mit dem ebenfalls hochmolekulare Assoziate bildenden α-Kristallin aus Wirbeltieren nachgewiesen werden (Ingolia & Craig, Proc.Natl.Acad.Sci. USA 79 (1982), 2360-2364). Auf DNA-Ebene sind Sequenzhomologien der kleinen Hsp's untereinander beschrieben.

Über die Funktion der ubiquitär vorkommenden kleinen Hsp's gibt es bislang nur wenige Daten. Erste Hypothesen sprechen von einer Rolle bei der Vermittlung der Thermotoleranz - dies konnte jedoch noch nicht eindeutig bewiesen werden (Landry et al., J. Cell Biol. 109 (1989), 7-15). Die starke Konservierung der Proteine, ihre ubiquitäre Verteilung, ihre streßbedingte Induktion sowie ihr maßgeblicher Anteil am Gesamtzellprotein lassen jedoch die Vermutung zu, daß die kleinen Hitzeschockproteine eine wesentliche, generelle Funktion in der Zelle besitzen.

Eine Stabilisierung von Proteinen durch Zusatz von unterschiedlichen, als Stabilisator wirkenden Proteinkomponenten ist bekannt. Die Nachteile bekannter Stabilisatorproteine bestehen jedoch darin, daß sie nur in Gegenwart von ATP wirksam sind, in großem molekularem Überschuß eingesetzt werden müssen oder/und selbst komplizierte instabile Oligomere darstellen.

Die Aufgabe der vorliegenden Erfindung bestand somit darin, ein neues Verfahren zur Stabilisierung von Proteinen in einer wäßrigen Lösung bereitzustellen, bei dem diese oben genannten Nachteile soweit wie möglich beseitigt sind.

Erfindungsgemäß wird die Aufgabe gelöst durch ein Verfahren zur Stabilisierung der Aktivität von Enzymen in einer wäßrigen Lösung, bei dem man der wäßrigen enzymhaltigen Lösung Hsp25 aus Maus und insbesondere rekombinantes Hsp25 zusetzt. Es wurde überraschenderweise festgestellt, daß Hsp25 aus Maus fähig ist, andere Proteine in wäßrigen Lösungen zu stabilisieren, wobei diese stabilisierende Wirkung insbesondere auf einer Verhinderung der Bildung von unlöslichen Aggregaten beruht.

Im erfindungsgemäßen Verfahren setzt man das Hsp25-Protein in einem molaren Verhältnis von 0,0001 : 1 bis 10 : 1 und insbesondere 0,001 : 1 bis 5 : 1, bezogen auf die zu stabilisierenden Proteine, ein.

Das erfindungsgemäße Verfahren kann bei beliebigen Proteinen angewandt werden, wobei es vorzugsweise bei solchen Proteinen angewandt wird, die wegen ihrer Temperaturempfindlichkeit oder aus sonstigen Gründen in gelöstem Zustand leicht zu Aggregation neigen. Ein wichtiges Anwendungsgebiet für das erfindungsgemäße Verfahren ist die Stabilisierung von Lösugnen, die enzymatisch aktive Proteine enthalten. Desgleichen sind mit dem erfindungsgemäßen Verfahren vorteilhaft auch wäßrige Lösungen von Antikörpern oder von Antikörpern abgeleiteten Derivaten (z.B. Fab- oder F(ab)₂-Fragmente) stabilisierbar.

In der Erfindung verwendet man Hsp25 aus Maus. Hsp25 bildet ein 32mer (Mr = 800 kD) (Behlke et al., FEBS Letters, Vol. 288 Nr. 1,2 (1991), 119-122), wobei die aktive Struktur (Monomer/Oligomer) nicht bekannt ist. Hsp25 in der Maus steht unter der Regulation eines Hitzeschock-Promotors, so daß durch Hitzestreß die Expression des Proteins induziert wird. Die Klonierung, Sequenzierung, Expression und Präparation von Hsp25 aus Maus in E.coli ist von Gaestel et al. in Eur. J. Biochem. 179 (1989), 209-213, J. Biol. Chem., Vol. 266, No. 22 (1991), S. 14721-14724, und in der DD-A1 273 071 beschrieben. Besonders vorteilhaft ist Hsp25 u.a. deshalb, da bezogen auf das 32mer der Protein-stabilisierende Effekt bereits bei einem deutlichen Unterschuß auf molarer Basis (das Optimum liegt bei etwa 0,5 mol Hsp25 pro 1 mol Protein) meßbar ist, zudem die Aktivität von enzymatisch aktiven Proteinen, wie z.B. Citratsynthase, in Gegenwart von Hsp25 unter Bedingungen, die eine Bindung des Hsp25 an Citratsynthase erlauben, nicht beeinflußt wird und Hsp25 extrem hitzeresistent ist, wobei nach Inkubation des Hsp allein bei 100°C über 15 Minuten und nachfolgender Abkühlung dessen Ausgangsaktivität wiedergefunden wurde. Hsp25 erscheint daher ein besonders vorteilhafter Kandidat für das erfindungsgemäße Verfahren. Durch die Möglichkeit der rekombinanten Herstellung ist es auch in ausreichenden Mengen verfügbar.

Eine bevorzugte Anwendung des erfindungsgemäßen Verfahrens liegt in der Unterstützung bei einer in vitro Faltung bzw. Renaturierung von denaturierten Proteinen, was wiederum ein weiterer Gegenstand der vorliegenden Erfindung ist. Die Anwendung wird vorzugsweise bei denaturierten Proteinen durchgeführt, die durch heterologe Expression in Prokaryonten synthetisiert worden sind und dabei im allgemeinen in Form von unlöslichen Inclusion Bodies anfallen. Vorzugsweise handelt es sich bei den heterolog exprimierten Proteinen um eukaryontische Proteine. Der Begriff "heterologe Expression" bedeutet in diesem Zusammenhang, daß ein Protein aus einem fremden (heterologen) Organismus oder/und unter Kontrolle eines fremden (heterologen) Promotors in einer Zelle, insbesondere einer prokaryontischen Zelle exprimiert und anschließend aus der Zelle oder dem Kulturmedium gewonnen wird. Diese heterologe Expression von Proteinen ist ein dem Fachmann auf dem Gebiet der Molekularbiologie bekanntes Standardverfahren, auf das hier nicht näher eingegangen werden muß. In diesem Zusammenhang wird z.B. auf die Darstellung der Expression klonierter Gene bei Sambrook et al. (Molekular Cloning. A Laboratory Manual, Cold Spring Harbor Laboratory Press (1989), Kapitel 16 und 17) verwiesen.

Ein weiterer Gegenstand der vorliegenden Erfindung beruht auf der Fähigkeit von Hsp25 aus Maus, native Proteine in gelöstem Zustand zu stabilisieren. So kann beispielsweise durch Zusatz von Hsp25-Protein eine Konstanthaltung der Empfindlichkeit bei optischen Tests in proteinhaltigen Lösungen erreicht werden, bei denen Störungen durch eine geringe Stabilität von in der Testlösung vorhandenen Proteinkomponenten auftreten können. Ein derartiger optischer Test beinhaltet vorzugsweise eine enzymatische Reaktion, besonders bevorzugt eine enzymatische Reaktion, bei der ATP als Cosubstrat vorhanden ist. Da die Stabilisierung der Proteine durch Hsp25 in Abwesenheit von ATP erfolgen kann, ist es auch ohne weiteres möglich, Proteinlösungen für enzymatische Tests zu stabilisieren, bei denen ATP als Cosubstrat eingesetzt wird, ohne daß dadurch ein störender Einfluß auf den enzymatischen Test ausgeübt wird.

Ein optischer Test im Sinne der vorliegenden Erfindung ist ein Bestimmungsverfahren, bei dem eine optische Größe oder die Änderung einer optischen Größe, z.B. Absorption, Transmission, Streulicht etc. gemessen wird. Vorzugsweise wird die vorliegende Erfindung bei Verfahren angewendet, in denen das Streulicht bestimmt wird, z.B. bei turbidometrischen, nephelometrischen und fluorimetrischen Verfahren. Eine derartige Anwendung des erfindungsgemäßen Verfahrens zur Stabilisierung von proteinhaltigen Lösungen ist daher ein wiederum weiterer Gegenstand der vorliegenden Erfindung. Besonders bevorzugt ist im Rahmen der Konstanthaltung der Empfindlichkeit von optischen Tests die zu stabilisierende Proteinkomponente α-Glucosidase PI.

Wiederum ein weiterer Gegenstand der vorliegenden Erfindung ist ein Reagenz zur Stabilisierung der Aktivität von Enzymen in wäßriger Lösung, welches rekombinantes Hsp25 aus Maus enthält, und zwar vorzugsweise in gelöster oder/und lyophilisierter Form.

Die Erfindung soll durch die folgenden Beispiele in Verbindung mit den Abbildungen weiter erläutert werden. Hierbei zeigt
- Abb. 1: die Aggregation der Citratsynthase bei 42°C in Gegenwart und Abwesenheit von aktivem Hsp25,
- Abb. 2: die Aggregation der α-Glucosidase bei 47,6°C in Gegenwart und Abwesenheit von aktivem Hsp25,
- Abb. 3: die Reaktivierungskinetik denaturierter α-Glucosidase in Gegenwart bzw. der Abwesenheit von aktivem Hsp25,
- Abb. 4: die Ausbeute an reaktivierter α-Glucosidase in Gegenwart und Abwesenheit von aktivem Hsp25 und
- Abb. 5: die Hitzeinaktivierung nativer α-Glucosidase PI während der Inkubation bei 46°C in Gegenwart und Abwesenheit von aktivem Hsp25.
- Abb. 6: den Einfluß von 15-minütiger Inkubation von Hsp25 bei 90°C auf die Wirkungsweise bei der Renaturierung denaturierter α-Glucosidase.

### Beispiele

Rekombinantes Hsp25 aus Maus wurde unter der Kontrolle des T7-Promotors in E.coli BL21 (DE3) exprimiert (Gaestel et al., Eur.J.Biochem. 179 (1989), 209-213) Nach Lyse der E.coli-Zellen wurde Hsp25 zunächst mit 35 % Ammoniumsulfat gefällt und mit einer DEAE-Anionenaustauschchromatographie aufgereinigt (NaCl-Gradient von 50 - 200 mmol/l NaCl) wie bei Gaestel, supra, beschrieben. Anschließend erfolgte eine Gelfiltration mit Superose 6 HR 10/30, bei der die Hsp25-Aggregate kurz nach dem Ausschlußvolumen eluierten. Hierzu wurde eine Säule mit 50 ml Puffer A (20 mM Tris/HCl, pH 7,6, 0,05 mM NaN₃, 2 µM PMSF, 0,5 mM DTE, 10 mM MgCl₂, 30 mM NH₄ CL) equilibriert, Hsp25 (maximal 2 mg in 200 µl Puffer A) injiziert und die Fraktionen unmittelbar nach dem Ausschlußvolumen der Säule gesammelt (Hsp25 existiert unter diesen Pufferbedingungen als Multimer mit einer molekularen Masse von etwa 800 kDa).

Citratsynthase aus Schweineherz (E.C.4.1.3.7) wurde von Boehringer Mannheim GmbH verwendet. Citratsynthase katalysiert die Kondensation von Acetyl-CoA und Oxalacetat zu Citrat. Dabei wird SH-CoA freigesetzt, und die enzymatische Aktivität der Citratsynthase indirekt durch die Reaktion der freien SH-Gruppen mit dem Thiol-Nachweisreagens Dithionitrobenzoesäure (DTNB) durch Messung der Absorption bei 412 nm verfolgt (Srere et al., Acta Chem.Scand. 17 (1963), 129-134).

α-Glucosidase PI (Maltase, E.C.3.2.1.20) (Boehringer Mannheim GmbH) aus Hefe wurde mit einer spezifischen Aktivität von > 130 U/mg verwendet. Die enzymatische Aktivität der α-Glucosidase wurde anhand der Freisetzung von p-Nitrophenol aus p-NPG durch die Änderung der Adsorption bei 405 nm bestimmt (Kopetzky et al., Mol.Gen. Genet. 216 (1989), 149-155).

Folgende Puffer wurden verwendet:

| | |
|---|---|
| Puffer A: | 50 mmol/l HEPES-KOH, pH 7,5 |

| | |
|---|---|
| Puffer B: | 50 mmol/l HEPES-KOH |
| | 50 mmol/l KCl, pH 7,0 |

| | |
|---|---|
| Lo-Puffer: | 40 mmol/l HEPES-KOH, pH 7,8 |
| | 20 mmol/l KCl |
| | 10 mmol/l (NH₄)₂SO₄ |
| | 2 mmol/l Kaliumacetat |

### Beispiel 1

### Einfluß von Hsp25 auf die Aggregation der Citratsynthase bei 42°C

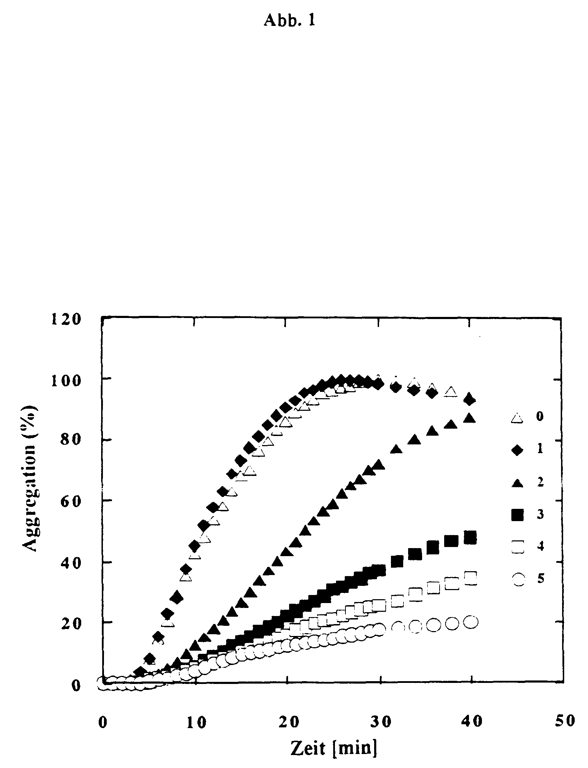

Aggregationsverlauf nativer Citratsynthase während der Inkubation bei 42°C: native Citratsynthase (30 µmol/l) wurde unter Rühren 1 : 200 in den auf 42°C vorinkubierten Lo-Puffer ± Hsp25 steigender Konzentrationen verdünnt und die Aggregation durch Messung der Lichtstreuung bei λem = 500 nm über die ersten 30 min. bei konstant 42°C erfaßt. Die Totzeit betrug etwa 3 s. Abb. 1 zeigt den Aggregationsverlauf unter Verwendung der folgenden Reaktionsansätze: (Gesamtvolumen: 1500 µl):

| | |
|---|---|
| 0. | Lo-Puffer |
| 1. | Lo-Puffer, 0,6 nmol/l Hsp25 |
| 2. | Lo-Puffer, 3,0 nmol/l Hsp25 |
| 3. | Lo-Puffer, 3,9 nmol/l Hsp25 |
| 4. | Lo-Puffer, 6,0 nmol/l Hsp25 |
| 5. | Lo-Puffer, 60 nmol/l Hsp25 |

### Beispiel 2

### Einfluß von Hsp25 auf die Aggregation der α-Glucosidase bei 47,6°C

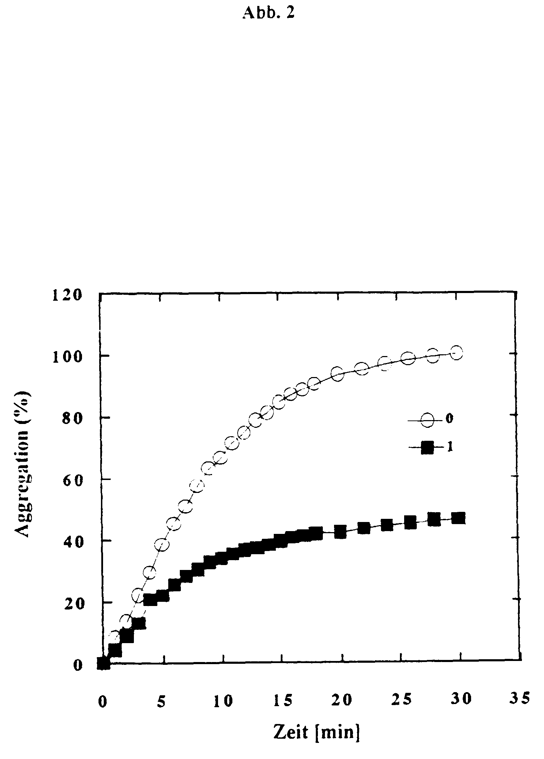

Aggregationsverlauf nativer α-Glucosidase während der Inkubation bei 47°C: native α-Glucosidase (4,1 µmol/l) wurde unter Rühren 1 : 75 in den auf 47,6°C vorinkubierten Puffer B ± Hsp25 (1,25 nmol/l) verdünnt und die Aggregation durch Messung der Lichtstreuung bei λex = 360 nm, λem = 360 nm über die ersten 30 min. bei konstant 47,6°C erfaßt. Die Totzeit betrug etwa 3 s. Abb. 2 zeigt den Aggregationsablauf für folgende Reaktionsansätze (1500 µl):

| | |
|---|---|
| 0. | Puffer B |
| 1. | Puffer B, 1,25 nM Hsp25 |

### Beispiel 3

### Einfluß von Hsp25 auf die Reaktivierung denaturierter α-Glucosidase

Es wurde eine Denaturierung der α-Glucosidase durch Inkubation von 9,4 µmol/l α-Glucosidase in 8 mol/l Harnstoff, 50 mmol/l Kaliumphosphat, 2 mmol/l EDTA, 20 mmol/l DTE, pH 7,5, für 2 h bei 20°C durchgeführt.
Anschließend erfolgte eine Renaturierung der α-Glucosidase in Abwesenheit bzw. Gegenwart von Hsp25. Dazu wurde denaturierte α-Glucosidase unter Rühren in einem Verhältnis von 1 : 100 in den Reaktivierungsansatz verdünnt (Endkonzentration: 0,094 µmol/l) und bei 20°C inkubiert. Nach definierten Zeitpunkten wurden dem Reaktionsansatz Aliquots entnommen und zur Bestimmung der Aktivität in den α-Glucosidase-Aktivitätstest eingesetzt.
Die Aktivität nativer α-Glucosidase nach Inkubation von 2 h bei 20°C wurde als 100 % gesetzt.

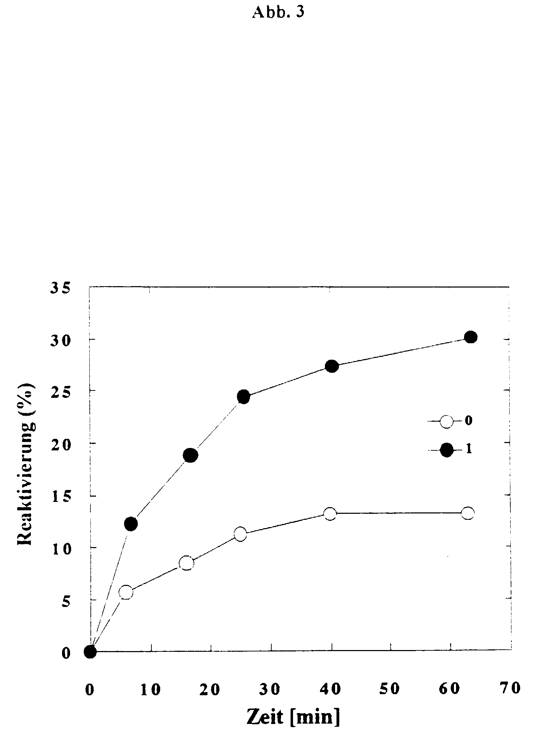

Abb. 3 zeigt die Ergebnisse der Reaktivierung mit und ohne Hsp25:

Reaktivierungsansätze (Gesamtvolumen: 300 µl):

| | |
|---|---|
| 0. | Puffer A |
| 1. | Puffer A, 0,05 µmol/l Hsp25 |

### Beispiel 4

### Einfluß von Hsp25 steigender Konzentrationen auf die Reaktivierung denaturierter α-Glucosidase

Die Denaturierung der α-Glucosidase erfolgte wie unter Beispiel 3 beschrieben.
Anschließend erfolgte eine Renaturierung der α-Glucosidase in Abwesenheit bzw. Gegenwart von Hsp25 steigender Konzentrationen. Dazu wurde denaturierte α-Glucosidase unter Rühren in einem Verhältnis von 1 : 100 in den Reaktivierungsansatz verdünnt (Endkonzentration: 0,094 µmol/l) und bei 20°C inkubiert.
Nach 2 h wurde dem jeweiligen Reaktionsansatz ein Aliquot entnommen und zur Bestimmung der Aktivität in den α-Glucosidase-Aktivitätstest eingesetzt.
Die Aktivität nativer α-Glucosidase nach Inkubation von 2 h bei 20°C wurde als 100 % gesetzt.

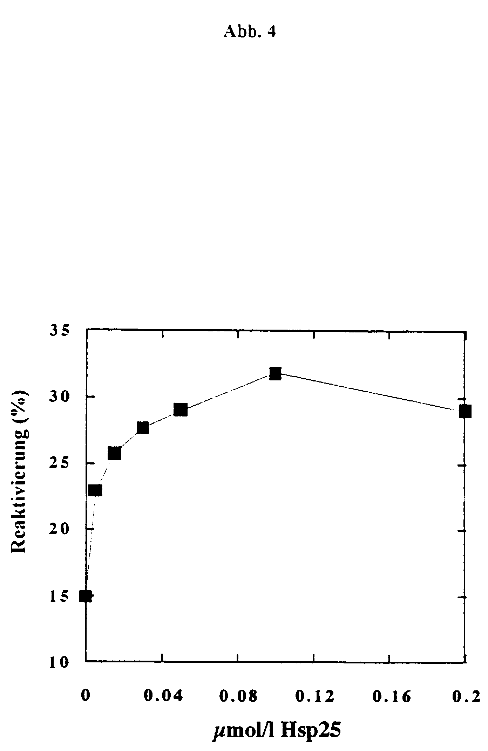

Abb. 4 zeigt die Ergebnisse für folgende Reaktivierungsansätze (Gesamtvolumen: 300 µl):

Puffer A in Abwesenheit und Gegenwart steigender Konzentrationen an Hsp25.

### Beispiel 5

### Einfluß von Hsp25 auf die Hitzeinaktivierung während der Inkubation bei 46°C

Hitzeinaktivierung nativer α-Glucosidase während der Inkubation bei 46°C: native α-Glucosidase (4,1 µM) wurde unter Rühren 1: 28 in den auf 46°C vorinkubierten Puffer B ± Hsp25 verdünnt (Endkonzentration: α-Glucosidase 0,15 µM) und bei konstant 46°C weiter inkubiert. Nach definierten Zeitpunkten wurden dem Reaktionsansatz jeweils Aliquots entnommen und zur Bestimmung der Aktivität in den α-Glucosidase-Aktivitätstest eingesetzt. Die Aktivität nativer α-Glucosidase bei 20°C wurde als 100 % gesetzt.

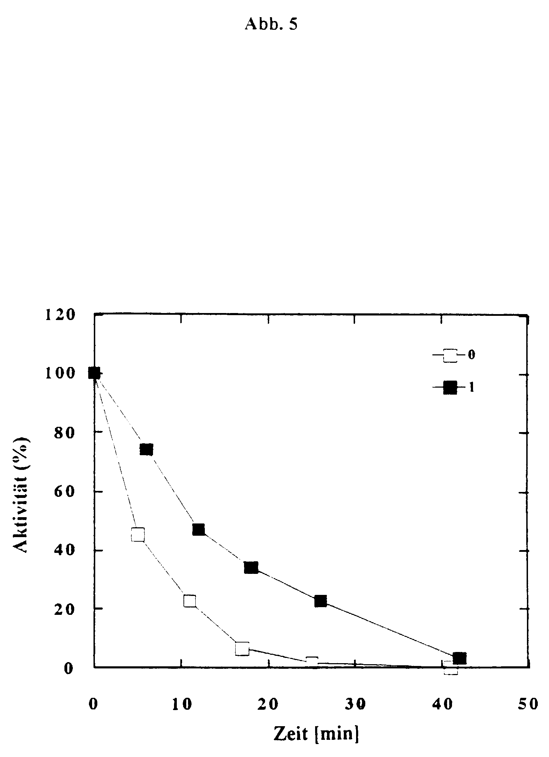

Abb. 5 zeigt die Ergebnisse für folgende Inkubationsansätze (Gesamtvolumen: 300 µl):

| | |
|---|---|
| 0. | Puffer B |
| 1. | Puffer B, 0,093 µmol/l |

### Beispiel 6

### Einfluß von Hsp25 auf die Reaktivierung der α-Glucosidase

Die Denaturierung der α-Glucosidase erfolgt wie unter Beispiel 3 beschrieben. Anschließend erfolgt eine Renaturierung der α-Glucosidase in Abwesenheit bzw. Gegenwart von Hsp25. Hsp25 wurde dazu entweder bei 20°C oder bei 90°C für 15 Minuten vorinkubiert, bevor es dem Renaturierungsansatz zugesetzt wurde. Die denaturierte α-Glucosidase wurde unter Rühren in einem Verhältnis von 1:100 in de Reaktivierungsansatz verdünnt (Endkonzentration : 0,094 µmol/l) und bei 20°C inkubiert. Zu definierten Zeitpunkten wurden dem jeweiligen Renaturierungsansatz Aliquots entnommen und in den Aktivitätstest eingesetzt. Die Aktivität nativer α-Glucosidase nach Inkubation von 2 Stunden bei 20°C wird als 100 % gesetzt.

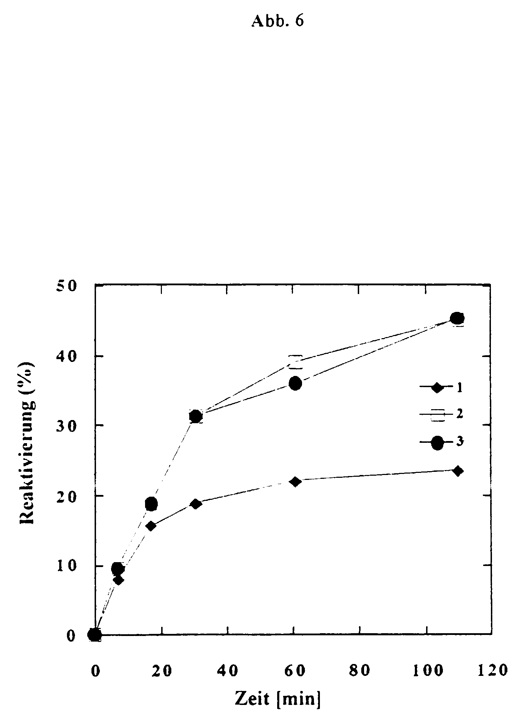

Abb. 6 zeigt die Ergebnisse für folgende Reaktivierungsansätze (Gesamtvolumen: 300 µl):

| | |
|---|---|
| 1. | Puffer A |
| 2. | Puffer A + 0,06 µM Hsp25 (inkubiert bei 20°C für 15 min.) |
| 3. | Puffer A + 0,06 µM Hsp25 (inkubiert bei 90°C für 15 min.) |

## Patentansprüche

1. Verfahren zur Stabilisierung der Aktivität von Enzymen in einer wäßrigen Lösung,
**dadurch gekennzeichnet**,
daß man der wäßrigen enzymhaltigen Lösung Hsp25 (Heat Shock Protein 25) aus Maus und insbesondere rekombinantes Hsp25 zusetzt.

2. Verfahren nach Anspruch 1,
**dadurch gekennzeichnet**,
daß man das Hsp25-Protein aus Maus in einem molaren Verhältnis von 0,0001 : 1 bis 10 : 1 und insbesondere von 0,001 : 1 bis 5 : 1 bezüglich der zu stabilisierenden Enzyme zusetzt.

3. Anwendung eines Verfahrens nach einem der Ansprüche 1 oder 2 zur Unterstützung bei der in vitro Renaturierung von denaturierten Enzymen.

4. Anwendung nach Anspruch 3,
**dadurch gekennzeichnet**,
daß es sich bei dem denaturierten Enzym um ein Enzym handelt, das durch heterologe Expression in Prokaryonten synthetisiert worden ist.

5. Anwendung eines Verfahrens nach einem der Ansprüche 1 oder 2 zur Konstanthaltung der Empfindlichkeit bei optischen Tests in enzymhaltigen Lösungen, bei denen Störungen durch eine geringe Stabilität von in der Testlösung vorhandenen Enzymkomponenten auftreten können.

6. Anwendung nach Anspruch 5,
**dadurch gekennzeichnet**,
daß der optische Test eine enzymatische Reaktion beinhaltet.

7. Anwendung nach Anspruch 5,
**dadurch gekennzeichnet**,
daß die zu stabilisierende Enzymkomponente α-Glucosidase PI ist.

8. Reagenz zur Stabilisierung der Aktivität von Enzymen in wäßriger Lösung,
**dadurch gekennzeichnet**,
daß es rekombinantes Hsp25 aus Maus enthält.

## Claims

1. Process for stabilizing the activity of enzymes in an aqueous solution,
**wherein**
Hsp 25 (heat shock protein 25) from the mouse and in particular recombinant Hsp 25 is added to the aqueous solution containing enzyme.

2. Process as claimed in claim 1,
**wherein**
the Hsp 25 protein from the mouse is added in a molar ratio of 0.0001 : 1 to 10 : 1 and in particular of 0.001 : 1 to 5 : 1 relative to the enzymes to be stabilized.

3. Use of a process as claimed in one of the claims 1 or 2 to support the in vitro renaturation of denatured enzymes.

4. Use as claimed in claim 3,
**wherein**
the denatured enzyme is an enzyme which has been synthesized by heterologous expression in prokaryotes.

5. Use of a process as claimed in one of the claims 1 or 2 to maintain a constant sensitivity in optical tests in solutions containing enzymes in which interferences due to a low stability of enzyme components present in the test solution can occur.

6. Use as claimed in claim 5,
**wherein**
the optical test includes an enzymatic reaction.

7. Use as claimed in claim 5,
**wherein**
the enzyme component to be stabilized is α-glucosidase PI.

8. Reagent for stabilizing the activity of enzymes in an aqueous solution,
**wherein**
it contains recombinant Hsp 25 from the mouse.

## Revendications

1. Procédé pour la stabilisation de l'activité d'enzymes dans une solution aqueuse,
caractérisé en ce que
on ajoute à la solution enzymatique aqueuse de la Hsp25 (Heat Shock Protein 25 - protéine de choc thermique 25) de la souris et en particulier la Hsp25 recombinée.

2. Procédé selon la revendication 1,
caractérisé en ce que l'on ajoute la protéine Hsp25 de souris dans un rapport molaire de 0,0001 : 1 jusqu'à 10 : 1 et en particulier de 0,001 : 1 à 5 : 1 par rapport à l'enzyme à stabiliser.

3. Application d'un procédé selon l'une des revendications 1 ou 2, afin d'assister la renaturation in vitro d'enzymes dénaturées.

4. Application selon la revendication 3,
caractérisée en ce que
pour l'enzyme dénaturée, il s'agit d'une enzyme qui a été synthétisée par expression hétérologue dans des procaryotes.

5. Application d'un procédé selon l'une des revendications 1 ou 2, pour maintenir constante la sensibilité lors de tests optiques dans des solutions enzymatiques, au cours desquels des perturbations peuvent se produire par suite d'une faible stabilité des composants enzymatiques présents dans la solution de test.

6. Application selon la revendication 5,
caractérisée en ce que
le test optique contient une réaction enzymatique.

7. Application selon la revendication 5,
caractérisée en ce que
le composant enzymatique à stabiliser est l'α-glucosidase PI.

8. Réactif pour la stabilisation de l'activité d'enzymes en solution aqueuse,
caractérisé en ce qu'
il contient une Hsp25 recombinée de souris.
